(19) Europäisches Patentamt

European Patent Office

Office européen des brevets

(11) Veröffentlichungsnummer: **0 324 414**
**A2**

(12) # EUROPÄISCHE PATENTANMELDUNG

(21) Anmeldenummer: **89100298.2**

(51) Int. Cl.⁴: **C07C 57/05 , C07C 57/055**

(22) Anmeldetag: **10.01.89**

(30) Priorität: **15.01.88 DE 3800991**

(43) Veröffentlichungstag der Anmeldung:
**19.07.89 Patentblatt 89/29**

(84) Benannte Vertragsstaaten:
**BE CH DE ES FR GB IT LI NL**

(71) Anmelder: **BASF Aktiengesellschaft**
**Carl-Bosch-Strasse 38**
**D-6700 Ludwigshafen(DE)**

(72) Erfinder: **Willersinn, Carl-Heinz, Dr.**
**Erich-Kaestner-Strasse 22**
**D-6700 Ludwigshafen(DE)**
Erfinder: **Martan, Hans Dr.**
**Mozartstrasse 62**
**D-6710 Frankenthal(DE)**
Erfinder: **Krabetz, Richard Dr.**
**Unterer Waldweg 8**
**D-6719 Kirchheim(DE)**
Erfinder: **Engert, Gerd-Juergen, Dr.**
**Muehlaustrasse 4**
**D-6700 Ludwigshafen(DE)**

(54) **Verfahren zur Herstellung von alpha, beta-monoolefinisch ungesättigten 3 bis 4 C-Atome enthaltenden Carbonsäuren durch Gasphasenoxidation.**

(57) Diese Erfindung betrifft ein Verfahren zur Herstellung von $\alpha,\beta$-monoolefinisch ungesättigten, 3 bis 4 C-Atome enthaltenden Carbonsäuren durch Gasphasenoxidation von 3 bis 4 C-Atome enthaltenden Alkenen und den entsprechenden $\alpha,\beta$-olefinisch ungesättigten Aldehyden mit hierfür üblichen oxidischen, Molybdän enthaltenden Katalysatoren in zwei Oxidationsstufen bei Drücken von 0,5 bis 10 bar, Abtrennen der ungesättigten Carbonsäuren nach der zweiten Oxidationsstufe durch Absorption und Rückführen von nichtumgesetzten Ausgangsstoffen, bei dem man den Druck nach der zweiten Oxidationsstufe auf 0,3 bis 1,0 bar einstellt.

EP 0 324 414 A2

# Verfahren zur Herstellung von α,β-monoolefinisch ungesättigten 3 bis 4 C-Atome enthaltenden Carbonsäuren durch Gasphasenoxidation

Diese Erfindung betrifft ein Verfahren zur Herstellung von α,β-monoolefinisch ungesättigten, 3 bis 4 C-Atome enthaltenden Carbonsäuren durch Gasphasenoxidation von 3 bis 4 C-Atome enthaltenden Alkenen und den entsprechenden α,β-olefinisch ungesättigten Aldehyden mit hierfür üblichen oxidischen, Molybdän enthaltenden Katalysatoren in zwei Oxidationsstufen.

Es ist aus einer Vielzahl von Literaturstellen bekannt, α,β-monoolefinisch ungesättigte, 3 bis 4 C-Atome enthaltende Carbonsäuren, wie besonders Acrylsäure, durch Gasphasenoxidation von 3 bis 4 C-Atome enthaltenden Alkenen, wie besonders Propylen, und den entsprechenden α,β-olefinisch ungesättigten Aldehyden, wie besonders Acrolein, herzustellen. Bei diesem bekannten Verfahren arbeitet man im allgemeinen in zwei Oxidationsstufen, wobei in einer ersten Oxidationsstufe zunächst das 3 bis 4 C-Atome enthaltende Alken, wie besonders Propylen, an einem Molybdän und zusätzlich weitere Metalle enthaltenden oxidischen Katalysator zu dem entsprechenden α,β-olefinisch ungesättigten Aldehyd, wie Acrolein, oxidiert und der Aldehyd dann in einer zweiten Oxidationsstufe gleichfalls an einem Molybdän enthaltenden von dem ersten Katalysator verschiedenen Katalysator zu der α,β-monoolefinisch ungesättigten Carbonsäure, wie besonders Acrylsäure weiter oxidiert wird. Nach den Angaben der US-PS 4 008 280 arbeitet man dabei im Druckbereich von 0,5 bis 10 Atm. Geeignete Molybdän enthaltende oxidische Katalysatoren sind z.B. in den US-PSen 4 008 280, 3 970 702 und 3 825 600 sowie in den DE-PSen 2 229 358 und 2 049 583 sowie in den DE-OSen 3 300 044 und 3 338 380 beschrieben. Bei den Katalysatoren der ersten Oxidationsstufe handelt es sich im wesentlichen um Wismut-Molybdat, das mit Oxiden anderer Metalle vermischt ist. Die oxidischen Katalysatoren der zweiten Oxidationsstufe enthalten zusätzlich zu Molybdän meist Vanadin und/oder Wolfram und/oder Antimon und/oder Kupfer. Die Katalysatoren werden in der Praxis als geformte Gebilde, z.B. Kugeln, Zylinderchen oder Ringe eingesetzt, die meist Durchmesser im Bereich von 3 bis 8 mm haben.

Derartige oxidische Katalysatoren sind bei den technischen Verfahren in einer Vielzahl parallel geschalteter Rohre mit freien Durchmessern von meist 1,5 bis 3 cm angeordnet, die sich in einem Wärmeaustauschmedium befinden. Die Rohre werden am Eingang des Reaktors mit einem gasförmigen Gemisch aus Alken, Sauerstoff, Stickstoff, geringen Mengen Wasserdampf sowie rückgeführten nicht umgesetzten Ausgangsstoffen beschickt. Die Umsetzung findet dann bei Temperaturen von 300 bis 450, insbesondere von 330 bis 420°C statt. Die heißen Reaktionsgase verlassen die zweite Reaktionsstufe und werden im allgemeinen einer Absorptionsstufe, beispielsweise einer Absorptionskolonne mit einem eventuell vorgeschalteten Vorkühlaggregat (Vorquensch) zugeführt. Dort werden Acrylsäure sowie gegebenenfalls nicht oxidierte, höhersiedende Nebenprodukte abgetrennt. Die nicht umgesetzten gasförmigen Ausgangsstoffe werden in das Abgas geleitet oder an den Anfang der ersten Oxidationsstufe zurückgeführt und das die Acrylsäure und Nebenprodukte enthaltende Absorbat destillativ aufgearbeitet. In einer derartigen Anlage wird das Ausgangsgasgemisch mit Verdichtern in die Rohre der ersten Oxidationsstufe und weiter durch die ganze Anlage gedrückt, wodurch am Anfang der Anlage meist Drucke im Bereich von 2 bis 2,5 bar, am Ende der zweiten Oxidationsstufe meist Drucke im Bereich von 1,4 bis 1,8 bar und am Ende der Absorptionsstufe meist Drucke im Bereich von 1 bis 1,2 bar herrschen.

Wie sich herausgestellt hat, wird jedoch, bedingt durch den Druckverlust der Gesamt-Anlage, insbesondere im ersten Reaktorabschnitt ein verhältnismäßig hoher Druck eingestellt. Hohe Drucke bei gleichbleibender Reaktionsgasmenge in der Eingangsschicht des Reaktors (1. Oxidationsstufe) führen aber zu einer unerwünscht schnellen Reaktion und zu einem erhöhten Temperaturmaximum in der Katalysatorschicht. Dies bedingt eine Minderung der Selektivität. Zur Verminderung von Druckverlusten hat man zwar schon spezielle Katalysatorformkörper, z.B. Ringe, eingesetzt, die einen verhältnismäßig geringen Druckverlust haben. Häufig sind jedoch Katalysator-Formkörper eines höheren Druckverlustes einfacher und damit preiswerter herzustellen und bieten zudem den Vorteil, in nahezu druckverlustfreien Schüttungen mit einer höheren Selektivität Wertprodukte zu produzieren als druckverlustarme Formkörper.

Es wurde nun gefunden, daß man α,β-monoolefinisch ungesättigte, 3 bis 4 C-Atome enthaltende Carbonsäuren durch Gasphasenoxidation von 3 bis 4 C-Atome enthaltenden Alkenen und den entsprechenden α,β-olefinisch ungesättigten Aldehyden mit hierfür üblichen oxidischen, Molybdän enthaltenden Katalysatoren in zwei Oxidationsstufen bei Drücken von 0,5 bis 10 bar, Abtrennen der ungesättigten Carbonsäuren nach der zweiten Oxidationsstufe durch Absorption und Rückführen von nicht umgesetzten Ausgangsstoffen mit Vorteil herstellen kann, indem man den Druck nach der zweiten Oxidationsstufe auf 0,3 bis 1,0 bar einstellt. Bei dem neuen Verfahren erhält man eine deutlich erhöhte Ausbeute am α,β-olefinisch ungesättigter Carbonsäure, insbesondere Acrylsäure. Das Einstellen des Drucks nach der zweiten Oxidationsstufe kann mit Vorteil derart vorgenommen werden, daß man die Reaktionsgase aus der zweiten Oxidationsstufe in bzw. durch die Absorptionsstufe saugt. Hierfür kann ein Verdichter unmittelbar nach der zweiten Oxidationsstufe oder mit Vorteil nach Abkühlen der heißen Reaktionsgase, beispielsweise nach einer Vorquenschappratur oder auch nach der Absorptionsstufe eingesetzt werden. Als Verdichter eignen sich beispielsweise Kreisel-, Kolben- oder Membranverdichter.

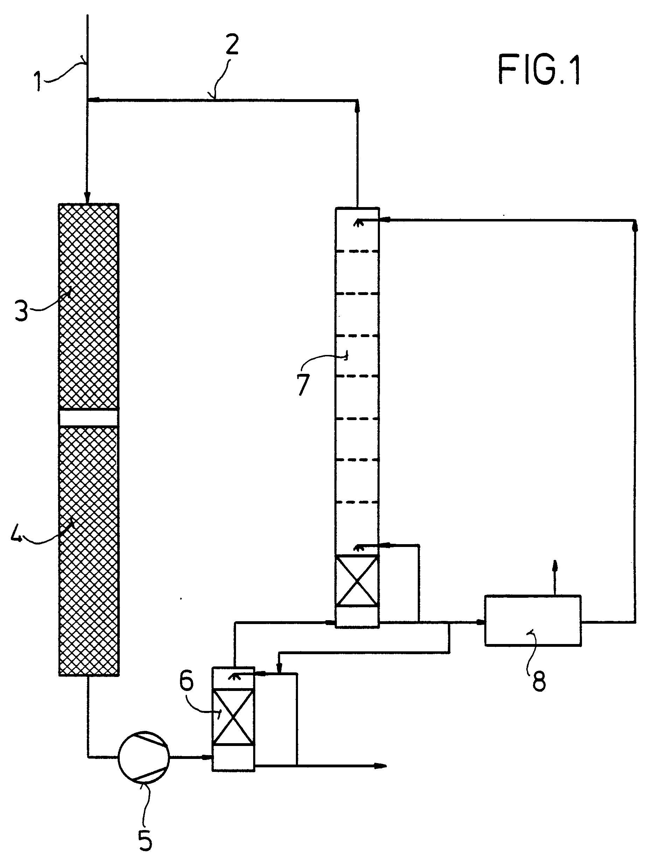

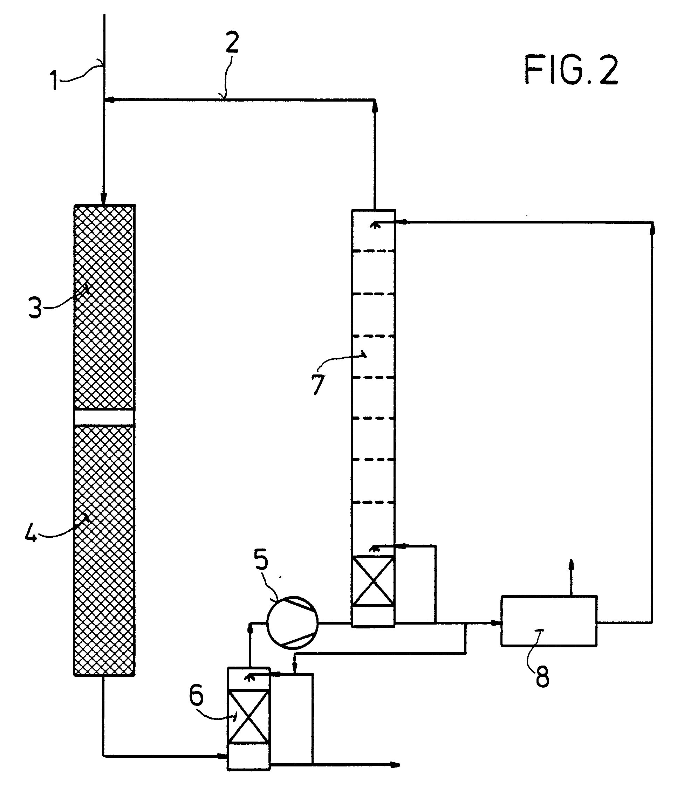

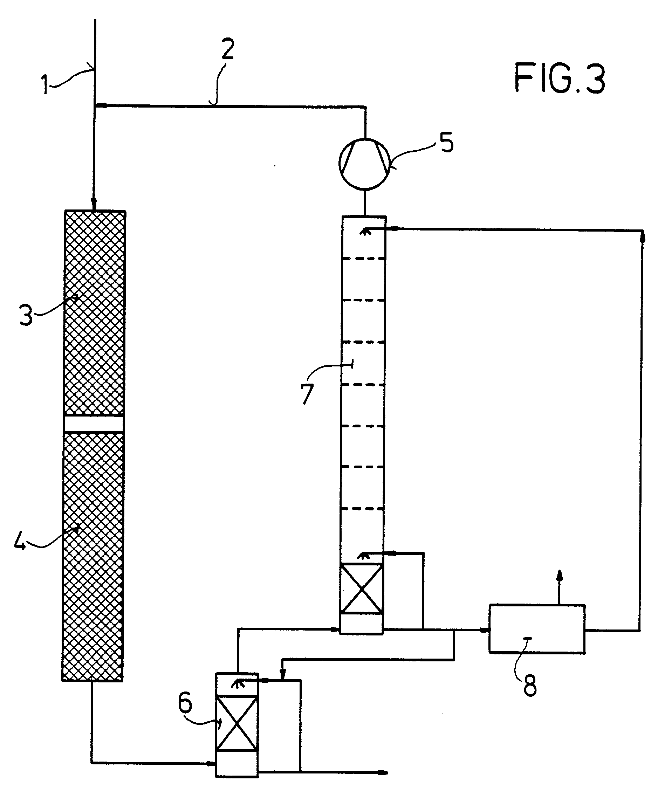

Die Anordnung eines derartigen Verdichters ist beispielhaft in den Figuren 1 bis 3 dargestellt. Diese zeigen schematisch den Oxidations- und Absorptionsteil einer Anlage zur Herstellung von Acrylsäure durch Oxidation von Propylen und Acrolein. Dabei wird das Frischgas (1) bestehend aus frischem Propylen, Luft und gegebenenfalls Wasserdampf nach dem Vermischen mit nicht umgesetzten Ausgangsstoffen (2) einer ersten Oxidationsstufe (3) und dann einer zweiten Oxidationsstufe (4) zugeführt. Die (4) verlassenden heißen Reaktionsgase werden dann einer Vorquentch-Apparatur (6) direkt (Figuren 2 und 3) oder über einen Verdichter (5) (Figur 1) zugeführt. In (6) kann das mit den hoch- bzw. nichtflüchtigen Verunreinigungen beladene Lösungsmittel ohne gesonderte Wärmezufuhr oder -abfuhr umgewälzt werden. Das Reaktionsgas belädt sich dort mit umgewälztem Lösungsmittel, und durch das Verdampfen von Lösungsmitteln kühlt es sich ab. Das abgekühlte Reaktionsgas wird dann direkt (Figuren 1 und 3) oder über einen Verdichter (5) (Figur 2) einer Absorptionskolonne (7) zugeführt in der Acrylsäure ausgewaschen wird. Diese verläßt die Absorptionskolonne (7) an deren unteren Ende und wird einer Destillationsanlage (8) zur Abtrennung des Lösungsmittels zugeführt. Das abgetrennte Lösungsmittel wird zur erneuten Absorptions von Acrylsäure auf den Kopf von (7) gegeben. Nicht umgesetzte Ausgangsstoffe (2), insbesondere Propen und Stickstoff sowie geringe Mengen Wasserdampf, CO, $CO_2$ und Acrolein verlassen (7) am oberen Ende und werden entweder ins Abgas geführt oder direkt (Figuren 1 und 2) bzw. durch den Verdichter (5) (Figur 3) dem Frischgas (1) zugeführt.

Für die Durchführung der folgenden Beispiele wird ein Modellreaktor verwendet, der in der DE-PS 2 513 405 beschrieben ist.

Beispiel 1

In den Modellrohrreaktor eines Rohr-Innendurchmessers von 25 mm werden 1100 cm³ Katalysator gemäß Beispiel 1 der DE-PS 23 38 111 für die erste Stufe und 1130 cm³ Katalysator gemäß Beispiel 1 der EP-PS 0 015 569 für die zweite Stufe eingefüllt. Bei einer Salzbadtemperatur von 360°C für die erste Stufe und von 279°C für die zweite Stufe wird der Reaktor mit stündlich 2400 N1 eines Gasgemisches aus 9 Mol.% Sauerstoff, 5 Mol.% Propen und einem Verdünnungsgas, das hauptsächlich aus Stickstoff sowie Spuren von CO, $CO_2$ und Acrolein besteht beschickt. Der Druck am Ende der zweiten Stufe wurde auf 0,95 bar eingestellt. Bei einem Propenumsatz von 97,4 Mol.% und einem Acroleinumsatz von 99,5 Mol.% erhält man eine Acrylsäureausbeute von 86,4 Mol.%.

Vergleichsversuch 1

Man arbeitet wie in Beispiel 1 angegeben, erhöht jedoch den Druck nach der zweiten Stufe auf 2 bar. Hierdurch steigt der Druck vor der ersten Stufe auf 2,25 bar an. Bei einer Salzbadtemperatur von 343°C für die erste Stufe und von 271°C für die zweite Stufe wird bei einem Propenumsatz von 97,4 Mol.% und einem Acroleinumsatz von 99,5 Mol.% eine Acrylsäureausbeute von nur 85,2 Mol.% erhalten.

Beispiel 2

Man arbeitet wie in Beispiel 1 angegeben, verwendet jedoch für die erste Stufe einen Katalysator gemäß Beispiel 1 der DE-OS 23 38 111, bei dem Indium durch Kalium ersetzt ist, und für die zweite Stufe einen Katalysator gemäß Beispiel 1 der EP-PS 0 015 569. Der Druck nach der zweiten Stufe wird auf 0,8 bar eingestellt. Bei einer Salzbadtemperatur von 347°C für die erste Stufe und von 280°C für die zweite Stufe wird bei einem Propenumsatz von 98,5 Mol.% und einem Acroleinumsatz von 99,2 Mol.% eine Acrylsäureausbeute von 88,5 Mol.% erhalten.

Vergleichsversuch 2

Man arbeitet wie in Beispiel 2 angegeben, stellt jedoch den Druck nach der zweiten Stufe auf 1,33 bar ein. Bei Badtemperaturen von 340°C für die erste Stufe und von 275°C für die zweite Stufe wird bei einem Propenumsatz von 98,3 Mol.% und einem Acoleinumsatz von 99,4 Mol.% eine Acrylsäureausbeute von 87,5 Mol.% erhalten.

**Ansprüche**

1. Verfahren zur Herstellung von $\alpha,\beta$-monoolefinisch ungesättigten, 3 bis 4 C-Atome enthaltenden Carbonsäuren durch Gasphasenoxidation von 3 bis 4 C-Atome enthaltenden Alkenen und den entsprechenden $\alpha,\beta$-olefinisch ungesättigten Aldehyden mit hierfür üblichen oxidischen, Molybdän enthaltenden Katalysatoren in zwei Oxidationsstufen bei Drücken von 0,5 bis 10 bar, Abtrennen der ungesättigten Carbonsäure nach der zweiten Oxidationsstufe durch Absorption und Rückführen von nicht umgesetzten Ausgangsstoffen, dadurch gekennzeichnet, daß man den Druck nach der zweiten Oxidationsstufe auf 0,3 bis 1,0 bar einstellt.

2. Verfahren nach Anspruch 1, dadurch gekennzeichnet, daß man die Einstellung des Drucks mit einem Verdichter vornimmt, der die Reaktionsgase aus der zweiten Oxidationsstufe in bzw. durch die Absorptionsstufe saugt.

EP 0 324 414 A2

FIG.1

345/87

FIG.2

FIG.3

345/87